(19) Europäisches Patentamt European Patent Office Office européen des brevets

(11) **EP 3 839 972 A1**

(12) **EUROPEAN PATENT APPLICATION**

(43) Date of publication:
**23.06.2021 Bulletin 2021/25**

(51) Int Cl.:
**G16H 50/20** (2018.01)       **A61B 5/085** (2006.01)
**A61M 16/00** (2006.01)

(21) Application number: **19383116.1**

(22) Date of filing: **16.12.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(71) Applicants:
- **Consorci Corporació Sanitària Parc Taulí**
  **08208 Sabadell (ES)**
- **Better Care, SL**
  **08203 Sabadell (ES)**

(72) Inventors:
- **SARLABOUS URANGA, LEONARDO**
  **08030 Barcelona (ES)**

- **AQUINO ESPERANZA, JOSE**
  **08037 Barcelona (ES)**
- **MAGRANS NICIEZA, RUDYS**
  **08030 Barcelona (ES)**
- **BLANCH TORRA, LLUIS**
  **08192 Sant Quirze del Vallés (ES)**
- **DE HARO LOPEZ, CANDELARIA**
  **08208 Sabadell (ES)**
- **LOPEZ AGUILAR, JOSEFINA**
  **08860 Castelldefels (ES)**

(74) Representative: **Clarke Modet & Co.**
**C/ Suero de Quiñones 34-36**
**28002 Madrid (ES)**

(54) **A DEVICE AND METHOD FOR RESPIRATORY MONITORING IN MECHANICALLY VENTILATED PATIENTS**

(57)     A device and method for respiratory monitoring in mechanically ventilated patients. The device comprises an input unit (120) for receiving a respiratory signal (112) comprising an airway pressure signal (106) and/or a flow signal (108) acquired during mechanical ventilation of a patient (102); and a data processing unit (130) configured to calculate (204) the sample entropy values (205) of a plurality of time-series data sets derived from each respiratory signal (112) to obtain (206) a sample entropy signal (208), extract (210) one or more features (212) from each sample entropy signal (208), compare (214) the features (212) with a baseline condition (216), and detect (218) a change in the breathing pattern and/or clusters of asynchronies based on the comparison.

The device allows to automatically, and personalized to each patient, detect complex patient-ventilator interactions (CP-VI) during mechanical ventilation of a patient.

Fig. 2

**Description**

*Field of the Invention*

The present disclosure relates to apparatuses and devices used for monitoring and assessing the respiratory activity in patients assisted by mechanical ventilators.

*Background of the Invention*

During mechanical ventilation two control systems of different nature coexist, namely the patient's brain center and the mechanical ventilator, using three different pumps (the machine pump, the inspiratory pump and expiratory pump). Ideally, these systems should work in harmony to effectively unload inspiratory muscles and provide a safe ventilation to enhance gas exchange and protect the lungs and the diaphragm. However, too often these systems do not work harmonically, in part because the natural breathing pattern variability may not be accompanied by most modes of mechanical ventilation, leading to asynchronous interactions. Besides, during disease states the respiratory drive could be altered by feedback signals (metabolic and chemical) from the cortex, jeopardizing patient-ventilator interactions.

Recent studies emphasize the role of asynchronies in outcomes. However, these studies focus on specific subtypes or Asynchorny Index (AI), as well as their distribution over time, which occurs in clusters within prolonged uneventful periods. Moreover, most of the studies are performed by visual analysis on the ventilator, and only a few of them are based on automated algorithms.

There are however some limitations when different types of asynchronies develop in a short period of time, or even overlapping between themselves. Moreover, sometimes patient's drive become evident only by an increase in the respiratory rate itself and this could be unnoticed or overestimated by the algorithms. Thus, an apparatus to assess irregularity and allow to automatically, and personalized to the patient, detect complex patient-ventilator interactions (CP-VI) during mechanical ventilation, including not only clusters of any kind of asynchronies but also changes in the breathing pattern, would be highly valuable.

*Description of the Invention*

The clinical value of irregularity during health and disease remains an issue of interest. Several researchers suggest that greater regularity is stronger associated with disease and compromised physiology, suggesting that irregularity at some extent could be normal, and might be useful as a physiological marker. Nonlinearity is a fundamental characteristic of normal physiologic data, given that a non-linear system is one whose behavior is not simply a summation of inputs into the structure; moreover, non-linear methods take into account the nonlinear physiologic response to a given stimuli. As such, this kind of approaches may provide insight into organ system interconnectivity, regulatory control, and complexity in a time series during disease states.

Entropy is a non-linear methods derived from theory of complex systems which measures the randomness and predictability of stochastic process, increasing with the increment of randomness. The present invention presents a novel, automated, personalized, non-invasive apparatus based on non-linear sample entropy analysis to detect CP-VI events using the entire airway pressure signal (Paw) and flow signal (Flow) during invasive mechanical ventilation.

The present invention relates to a device and a method for respiratory monitoring in mechanically ventilated patients. The device comprises an input unit for receiving one or more respiratory signals acquired during mechanical ventilation of a patient, the respiratory signals comprising at least an airway pressure signal and/or a flow signal from a mechanical ventilator. The device further comprises a data processing unit that computes, for each respiratory signal, the sample entropy values of a plurality of time-series data sets derived from the respiratory signal, obtaining a sample entropy signal. The data processing unit extracts at least one feature from each sample entropy signal and compares the extracted features with a baseline condition to detect a change in the breathing pattern and/or clusters of asynchronies.

In an embodiment, the data processing unit is configured to extract features from each sample entropy signal over a plurality of analysis time intervals. The data processing unit may be configured to assign the baseline condition to the at least one feature extracted on a first analysis time interval and, for each analysis time interval, if at least one feature extracted on the analysis time interval is lower than the corresponding feature assigned to the baseline condition, update the baseline condition with said lower extracted feature.

In an embodiment, the data processing unit is configured to acquire the plurality of time-series data sets derived from each respiratory signal using time sliding windows. The data processing unit may be configured to obtain each sample entropy signal by applying smoothing on the corresponding sample entropy values. The at least one feature extracted from each sample entropy signal preferably comprises any of the following: a maximum value, a mean value, a cumulative sum value, or a combination thereof.

The data processing unit is preferably configured to calculate, for each extracted feature, an increase in the extracted feature relative to a corresponding feature of the baseline condition, and detect a change in the breathing pattern and/or clusters of asynchronies if the increase exceeds a threshold.

According to an embodiment, the respiratory signal comprises a flow signal, the sample entropy values are calculated using an embedding dimension m equal to 3 and a tolerance *r* comprised in the interval [0.1xSD-0.3xSD], the at least one feature extracted from the sample entropy signal associated to the flow signal is the maximum value, and the threshold is comprised in the interval [20%-30%].

According to another embodiment, the respiratory signal comprises an airway pressure signal, the sample entropy values are calculated using an embedding dimension m equal to 3 and a tolerance *r* comprised in the interval [0.1xSD-0.2xSD], the at least one feature extracted from the sample entropy signal associated to the airway pressure signal is the maximum value, and the threshold is comprised in the interval [40%-50%].

The device may further comprises an alarm unit for emitting an alarm when a change in the breathing pattern and/or clusters of asynchronies is detected.

In accordance with a further aspect of the present invention there is provided a method for respiratory monitoring in mechanically ventilated patients. The method comprises the following steps: receiving at least one respiratory signal comprising an airway pressure signal and/or a flow signal acquired during mechanical ventilation of a patient; for each respiratory signal, calculating the sample entropy values of a plurality of time-series data sets derived from the respiratory signal, obtaining a sample entropy signal; extracting at least one feature from each sample entropy signal; comparing the at least one feature with a baseline condition; and detecting a change in the breathing pattern and/or clusters of asynchronies based on the comparison. The method may comprise any other additional step performed by the device, as previously described.

In accordance with yet a further aspect of the present invention there is provided a computer program product for respiratory monitoring in mechanically ventilated patients. The computer program product comprises at least one computer-readable storage medium comprising a set of instructions stored therein which, when executed by a processor, causes the processor to: receive at least one respiratory signal comprising an airway pressure signal and/or a flow signal acquired during mechanical ventilation of a patient; for each respiratory signal, calculate the sample entropy values of a plurality of time-series data sets derived from the respiratory signal, obtaining a sample entropy signal; extract at least one feature from each sample entropy signal; compare the at least one feature with a baseline condition; and detect a change in the breathing pattern and/or clusters of asynchronies based on the comparison. The computer-readable storage medium may comprise additional instructions causing the processor to execute any other additional step performed by the device, as previously described.

The detection performed by the device is obtained with high sensitivity, specificity and accuracy, irrespective of the ventilation mode; e.g. patients ventilated in PSV (pressure support ventilation), PCV (pressure assist-control ventilation), VCV (volume assist-control ventilation) modes. The device relies on the non-linear analysis of sample entropy to measure the irregularity and randomness of the entire physiological signals of Paw and Flow, in order to detect automatically and personalized from patients own's interaction with the ventilator when those change and become complex. Moreover, since the accuracy of the device is not influenced by the ventilation mode, the device could be applied to the most commonly used ventilation modes in critically ill patients.

The device can be used in different clinical situations in order to assess the clinical meaning of variability and complexity of patient-ventilator interactions during mechanical ventilation; for instance:

- During weaning trials. For instance, since it has been proved that spontaneous breathing trials (SBT) performed during 30 minutes in PSV lead to significantly higher rates of successful extubation compared to 2 hours of T-piece), the device can be used in this situation to provide an insight into patient-ventilator interactions during this phase of ventilatory support and study the relationship and clinical implication of CP-VI with success or failure of SBT.

- Before self-extubation, aiming to study how patients interact with the ventilator during a time-frame prior to a voluntary removal of the endotracheal tube.

- A monitoring tool to guide adjustments to the ventilatory setting.

- For developing smart and predictive alarms.

*Brief Description of the Drawings*

A series of drawings which aid in better understanding the invention and which are expressly related with an embodiment of said invention, presented as a non-limiting example thereof, are very briefly described below.

Figure 1 represents the components of a device according to an embodiment of the present invention.

Figure 2 illustrates the operations performed by the data processing unit of the device.

Figure 3 depicts a flow diagram of the data processing unit operations according to a particular embodiment.

Figure 4 shows the sample entropy feature extraction process applied by the device to a respiratory signal.

Figures 5A, 5B and 5C represent three different analysis time intervals of Flow and Paw signals acquired during mechanical ventilation of a patient.

Figure 6 represents a flowchart for the optimization procedure and validation of the sample entropy parameters and settings.

Figure 7 show the results of the optimization process of Figure 6.

Figure 8 depicts the performance statistics obtained using the optimized parameters.

*Description of a Preferred Embodiment of the Invention*

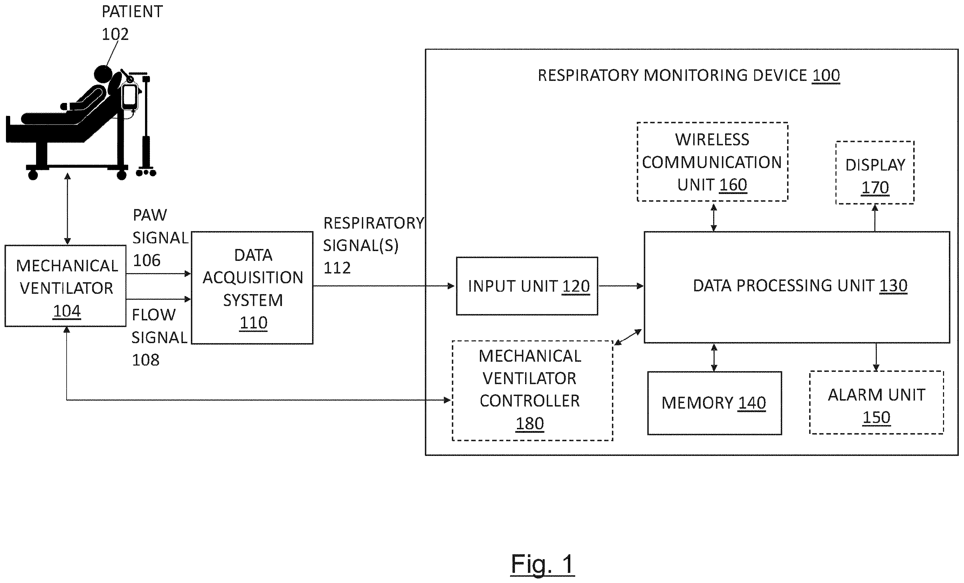

The present invention refers to a device for respiratory monitoring in mechanically ventilated patients. **Figure 1** shows a device 100 according to an embodiment of the present invention, and the interaction of the device with the mechanical ventilator of a patient.

Figure 1 depicts a patient 102 assisted by a mechanical ventilator 104. The output of the mechanical ventilator 104 normally includes, at least, an airway pressure signal 104 (Paw) and a flow signal (Flow) 108. A data acquisition system 110 acquires said output signals with a certain sampling frequency, e.g., 200 Hz. Paw and Flow signals can be continuously recorded using a dedicated software (e.g., Better Care™) using drivers specifically designed to interact with the output signal of mechanical ventilators and bedside monitors. Recorded signals can be synchronized and stored for further analysis.

The data acquisition system 110 provides an input unit 120 of the device 100 with at least one respiratory signal 112 comprising the sampled airway pressure signal and/or the sampled flow signal. For instance, the respiratory signal 112 may comprise only the flow signal 108 conveniently sampled. A data processing unit 130 of the device 100 may carry out further subsampling of the respiratory signals 112. For instance, respiratory signals 112 can be decimated at a sampling rate of 100 Hz. A memory 140 is used to store data in the calculations performed by the data processing unit 130. The memory 140 may be an entity external to the data processing unit 130, as depicted in Figure 1. Alternatively, the memory 140 may be an internal element included in the data processing unit 130.

Optionally, the mechanical ventilator 104 may be directly connected to the device 100. In that case, the data acquisition system 110 would be included in the device 100 itself.

The respiratory monitoring device 100 normally monitors the patient's respiratory signals in real time, at the same time as the signals are captured (i.e., an on-the-fly mode). In another embodiment, the respiratory signals 112 are previously stored by the data acquisition system 110 and later on analysed by the device 100 (i.e., an off-line mode).

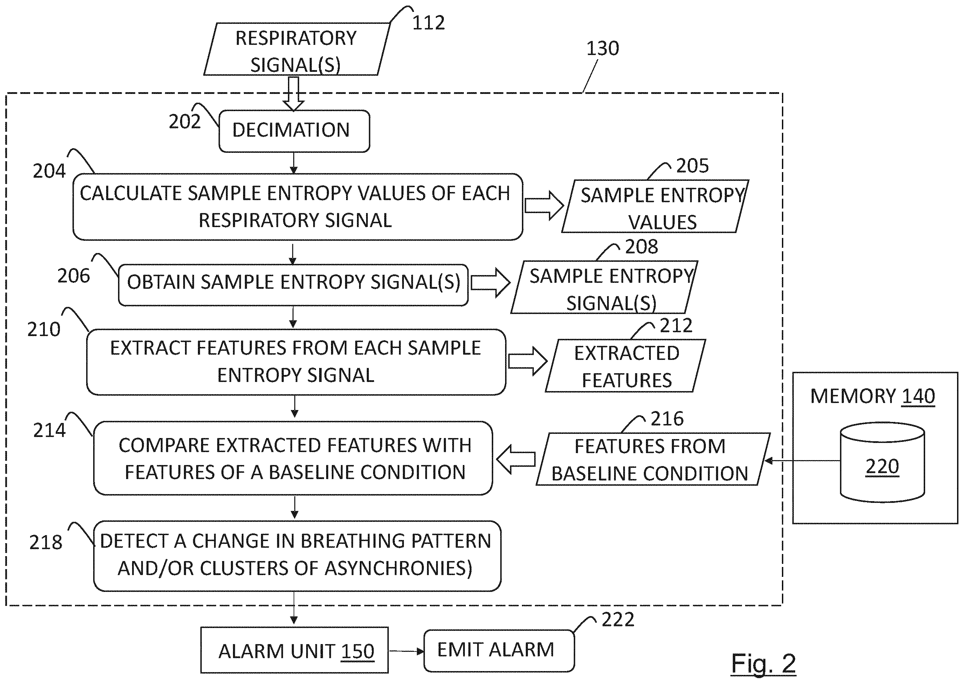

**Figure 2** depicts, according to an embodiment, the operations performed by the data processing unit 130 upon reception of the one or more respiratory signals 112. Each respiratory signal may be independently decimated 202 at a determined sampling rate.

Alternatively, the respiratory signals 112 may be already conveniently sampled by the external data acquisition system 110, rendering the decimation unnecessary. Thereafter, the data processing unit 130 calculates 204 sample entropy values 205 of a plurality of time-series data sets of each respiratory signal 112.

Sample entropy (SE) is a non-linear measure that allows assessing the randomness of a series of data. The main advantage of sample entropy is its consistency against short-length and noisy medical time series. The sample entropy calculation requires three parameters: the embedding dimension m (a positive integer), the tolerance value or similarity criterion r (a positive real number), and the total length N of the analysed series. The sample entropy is defined as the negative logarithm of the conditional probability that two sequences of patterns of m consecutive samples, which are similar to each other within a tolerance *r*, will remain similar when one consecutive sample is added (*m* + 1), excluding self matches, and it is calculated as follows (as defined for instance in Richman, J. S. & Moorman, J. R. "Physiological time-series analysis using approximate entropy and sample entropy", American Journal of Physiology - Heart and Circulatory, 2000):

$$SE(m, r, N) = -ln\left(\frac{A^m(r)}{B^m(r)}\right) \quad (1)$$

Where $B^m(r)$ and $A^m(r)$ are defined as:

$$B^m = \frac{1}{N-m} \sum_{i=1}^{N-m} B_i^m(r) \qquad (2)$$

$$A^m = \frac{1}{N-m} \sum_{i=1}^{N-m} A_i^m(r) \qquad (3)$$

So that, $B^m(r)$ represents the probability that two sequences will match for m samples, whereas $A^m(r)$ represents the probability that two sequences will match for m + 1 samples, such that the distance between two vectors $d[X(i), X(j)] \leq r$:

$$B_i^m(r) = \frac{B_i(r)}{N-m-1} \qquad (4)$$

$$A_i^m(r) = \frac{A_i(r)}{N-m-1} \qquad (5)$$

The m parameter is generally taken as 2 while $r$ parameter is normally ranging between 0.1 to 0.25 times the standard deviation of the series length.

For each respiratory signal, an associated sample entropy signal 208 is obtained 206. A sample entropy signal 206 may be a discrete-time signal (i.e. a time series) formed by the calculated sample entropy values 205. Alternatively, a sample entropy signal 208 may be a continuous signal obtained from the sample entropy values 205; for instance, by applying a filtering or a smoothing algorithm on the sample entropy values 205.

Features 212 are then extracted 210 from each sample entropy signal 208. The extracted features may include, for instance, a maximum entropy value, a mean entropy value, a cumulative sum entropy value or a combination thereof. The extracted features 212 are compared 214 with features corresponding to a baseline condition 216 of the patient 102, which may be retrieved from a database 220 stored on the memory 140 or even established and updated during the analysis.

Depending on the result of the comparison, the data processing unit 130 is able to detect 218 if a change in the breathing pattern of the patient 102 and/or clusters of asynchronies have occurred. In an embodiment, the detection is reported to an alarm unit 150 of the device 100 configured to emit an alarm 222, such as sending or displaying a warning message or activating an alarm sound. The alarm unit 150 may be an entity external to the device 100. In another embodiment, the data processing unit 130 is configured to emit an alarm, for instance by sending a warning message to an external device (e.g., an external alarm unit) using a wireless communication unit 160 of the device 100, and/or by displays a warning message on a display 170 of the device 100. In yet another embodiment, the data processing unit 130 may send instructions to a mechanical ventilator controller 180 (either incorporated in the device 100 itself or external to the device 100) to control the operation of the mechanical ventilator 104 in response to the detection made (e.g. adjust parameter settings and/or change the ventilation mode). The modules and units of the device 100 depicted in dotted lines in Figure 1 are optional elements of the device.

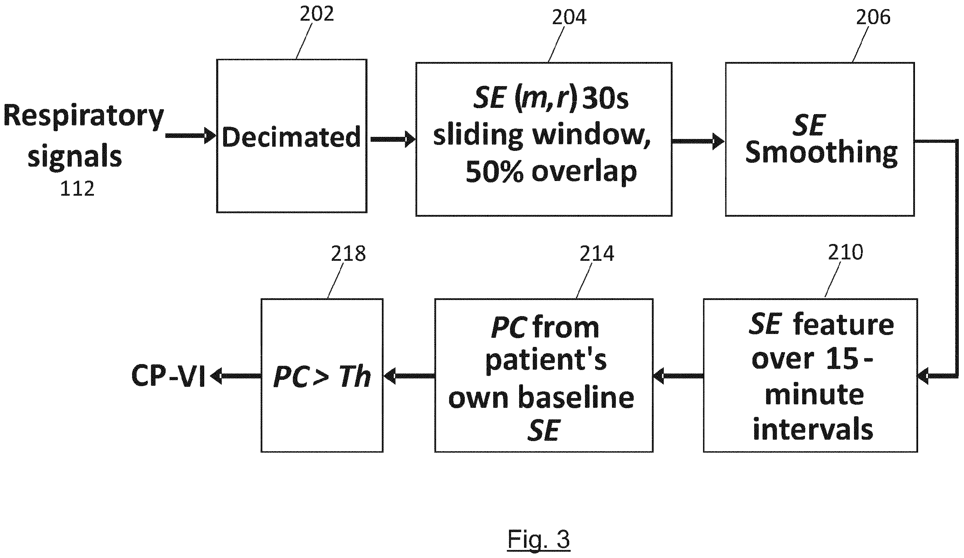

The flow diagram depicted in **Figure 3** refers to a particular embodiment in which respiratory signals 112 (flow and Paw signals) are decimated 202 at a sampling rate of 100 Hz and time-series data sets are acquired from each respiratory signal using time sliding windows (e.g., 30s overlapping sliding windows, with a 50% overlap between consecutive windows). For each time-series data set included in a time sliding window, a sample entropy value 205 is calculated 204 for determined values of embedding dimension m and tolerance $r$, thereby obtaining a time-series data set of sample entropy values 205 for each respiratory signal 112 received.

A smoothing filter is then applied to each entropy time-series data set to obtain 206 a sample entropy signal 208 for each respiratory signal 112; for instance, an exponential moving average of 8-period length may be used to reduce noise and better expose the consistency of each sample entropy signal 208. Features are extracted 210 from each sample entropy signal 208 over a plurality of analysis time intervals of a certain duration (e.g., 15-minute intervals). In an embodiment, three features are extracted (e.g., derived or computed) from each 15-minute interval of the sample entropy signal 208: the cumulative sum, the mean and the maximum of the sample entropy values within the analysis time interval.

The extracted features are compared 214 with features from a baseline condition. In an embodiment, a percent of change (PC) is computed for each extracted feature relative to a patient's own baseline value. For instance, if the value of the extracted feature (e.g. the maximum value of the sample entropy signal) in an analysis time interval is 0.12 and the baseline value established for said feature is 0.1, the percent of change would be 20% for said analysis time interval.

Based on the comparison, a change in the breathing pattern of the patient and/or clusters of asynchronies can be detected 218. In the example, the percent of change (PC) is compared with a determined threshold (Th). A complex patient-ventilator interaction (CP-VI) is detected when the percent of change (PC) exceeds (or equals) the threshold (Th). CP-VI is defined as the presence, alone or in combination, of a change in the breathing pattern (an increment in respiratory rate of more than 50%) and/or the occurrence of any kind of asynchronies of more than 30% in 3 minutes (ineffective expiratory effort, double cycling, premature cycling, prolonged cycling and reverse triggering), without discerning from one another.

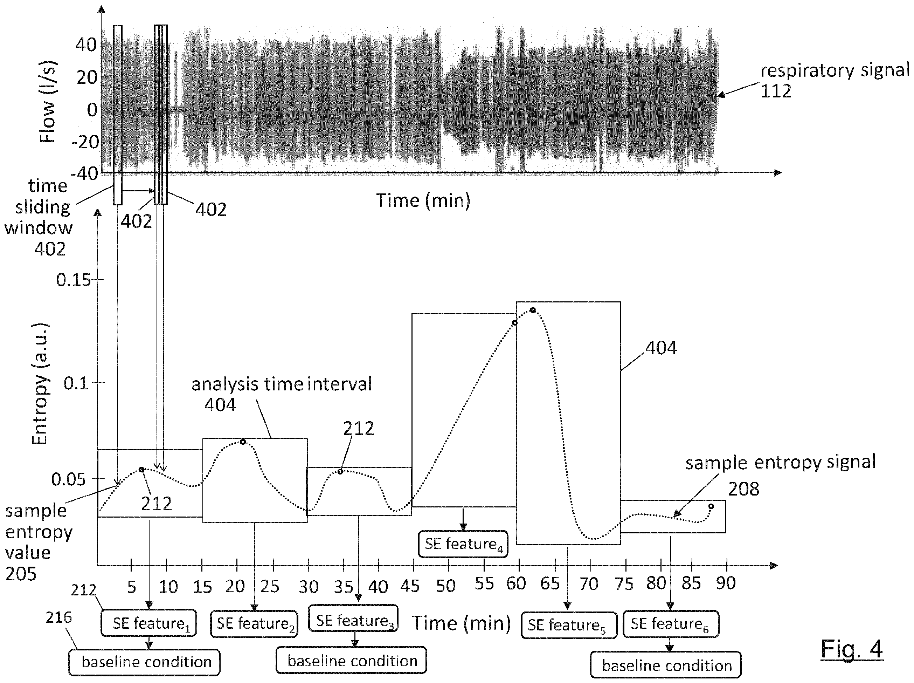

**Figure 4** depicts an exemplary embodiment to obtain the sample entropy signal 208 and the features extracted thereof. In the example, the respiratory signal 112 only includes the flow signal (in I/s), decimated at a sampling rate of 100 Hz. A sample entropy value 205 is calculated for each time-series data set of the respiratory signal 112 included within a time sliding window 402. In the example, the time sliding window 402 is a 30-second time window, the number of data points N of each time series data set is 3000 (considering the size of the window of 30 seconds and the sample rate of 100 Hz), and the parameters used for computing the sample entropy values 205 are m=3 and r=0.2xSD (standard deviation). Different parameters (e.g., sampling frequency, size of the time sliding window 402, *m*, *r*) may be used.

The time sliding windows 402 may be overlapping windows. In the example, the time sliding windows 402 overlap each other 50%, so that the first time sliding window 402 covers the interval [0s-30s], the second time sliding window 402 covers the interval [15s-45s], the third time sliding window 402 covers the interval [30s-60s], and so on, thereby having a sample entropy value 205 computed each 15 seconds.

In the example, the time-series computed sample entropy values 205 define a sample entropy signal 208. The sample entropy signal 208 may also be obtained by applying a smoothing algorithm on the time-series sample entropy values 205. One or more features are extracted from the sample entropy signal 208. In the example, only one feature is extracted (SE feature), the maximum value. The feature is repeatedly extracted over a plurality of analysis time intervals 404 of a certain duration (for instance, 15-minute intervals in the embodiment of Figure 4). In this case, since there is one sample entropy value 205 computed each 15 seconds, every analysis time interval 404 includes 60 sample entropy values.

The sample entropy signal includes at least one analysis time interval 404, and therefore at least one feature is extracted. In the example, the sample entropy signal 208 is 90-minute long and includes six analysis time intervals 404, thereby obtaining six sample entropy features (SE feature$_1$ to SE features), one for each interval. In the example, the feature 212 extracted for each analysis time interval 404 is the maximum value (highlighted with a circle) of the sample entropy values 205 contained within the corresponding analysis time interval 404. Other features may be used, alone or in combination, such as the mean value in the interval, or the cumulative cumulative sum value.

Each sample entropy feature 212 extracted in an analysis time interval 404 is compared with a corresponding feature of a baseline condition. For instance, if a mean value and a maximum value are extracted (two features), they are compared with corresponding features (mean value and maximum value) of the baseline condition. The baseline condition may be predefined for the patient 102, or it may have been obtained in previous analyses. In an embodiment, the baseline condition is assigned to the sample entropy feature (or features, should there be more than one) extracted on the first analysis time interval 404. For instance, in the example of Figure 4 the baseline condition assigned to patient 102 is SE feature$_1$, the feature (maximum value) extracted in the first interval. If more than one feature is extracted in each interval analysis time interval 404, the features extracted in the first interval may be assigned as the corresponding features for baseline condition. After extracting a feature in each analysis time interval 404, the data processing unit 130 may check if the value of said feature is lower than the value of the corresponding feature assigned to the baseline condition, and in case update the baseline condition with the lower extracted feature. In the example of Figure 4 the feature extracted in the third analysis time interval 404 (SE features) is lower than the assigned baseline condition (in this case, SE feature$_1$), and the baseline condition is conveniently updated with the value of SE features. Similarly, the baseline condition is also updated with the extracted feature of the sixth analysis time interval 404 (SE features), since it is lower than the latest baseline condition (SE features).

According to the embodiment of Figure 3, a percent of change (PC) between the extracted feature and the baseline condition may be computed. If the increase in the extracted feature relative to the corresponding feature of the baseline condition exceeds a threshold (Th), a change in the breathing pattern and/or clusters of asynchronies is detected for the corresponding analysis time interval 404. In the example of Figure 4, Th is set to 25% and the percent of change for each interval is the following:

- First analysis time interval. SE feature$_1$= 0.055. Baseline condition= 0.055. PC=0%. No detection (PC<Th).

- Second analysis time interval. SE feature$_2$= 0.066. Baseline condition= 0.055. PC=20%. No detection (PC<Th).

- Third analysis time interval. SE feature$_3$= 0.053. Baseline condition= 0.055. PC=-4%. No detection (PC<Th). Baseline condition updated to 0.053.

- Forth analysis time interval. SE feature$_4$= 0.13. Baseline condition= 0.053. PC=145%. CP-VI detected (PC>Th).

- Fifth analysis time interval. SE feature$_5$= 0.135. Baseline condition= 0.053. PC=155%. CP-VI detected (PC>Th).

- Sixth analysis time interval. SE feature$_6$= 0.03. Baseline condition= 0.053. PC=-43%. No detection (PC<Th). Baseline condition updated to 0.03.

In this example two CP-VI situations are detected, in the fourth and fifth intervals.

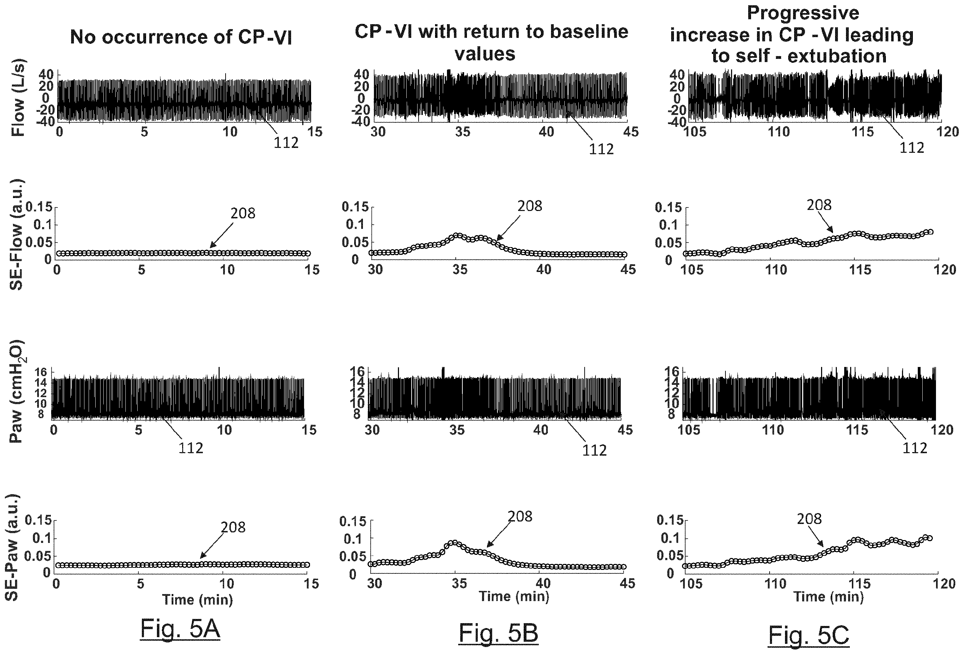

**Figures 5A, 5B** and **5C** depict three different analysis time intervals (each one of 15-minute length) of Flow and Paw signals acquired during mechanical ventilation of a patient. The derived sample entropy (SE) tracing over the Flow and Paw is also shown. In this example the sample entropy is calculated using m=3, r=0.2xSD of each overlapping sliding window 402 of 30s, as explained in Figure 4. Three different CP-VI events are represented corresponding to: absence of CP-VI (Figure 5A), presence of CP-VI that later returned to baseline values (Figure 5B), and progressive increase in CP-VI leading to self-extubation episode (Figure 5C). As shown in these figures, the sample entropy signal 208 is very sensitive to irregularity developed during mechanical ventilation.

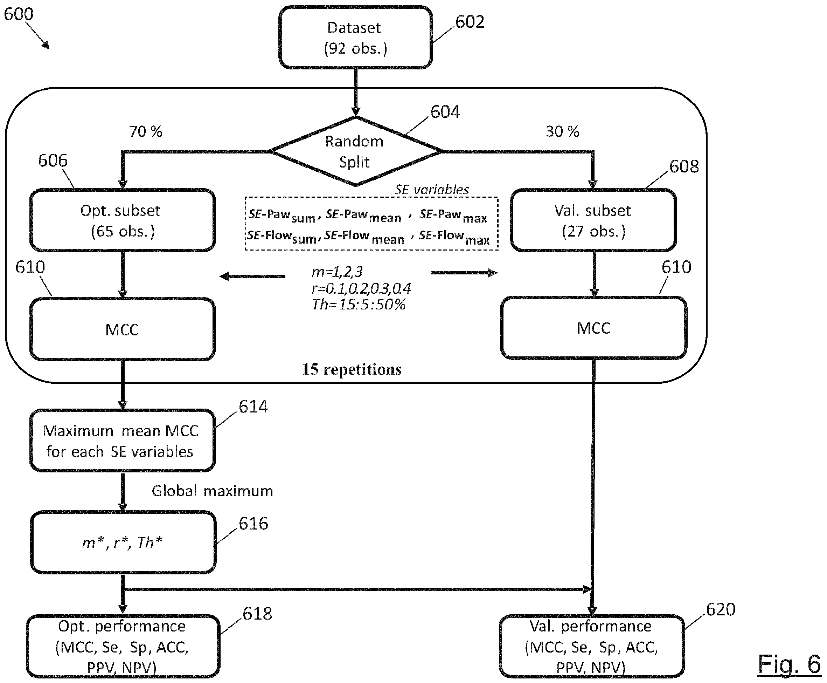

**Figure 6** represents an optimization procedure 600 to select the optimal sample entropy settings (values of m and r) and the optimal value of threshold of change (Th), on each SE derived features (SE-Flow$_{sum}$, SE-Flow$_{mean}$, SE-Flow$_{max}$, SE-Paw$_{sum}$, SE-Paw$_{mean}$, and SE-Paw$_{max}$), which also allows selecting the optimal respiratory signal (Flow, Paw) and the optimal SE feature (cumulative sum value, maximum value, mean value) for robustly estimating CP-VI. In the optimization procedure 600, a dataset 602 (in the example formed by 92 observations of 15 minutes each) visually validated by experts is randomly divided 604 into two subsets: an optimization subset 606 (e.g., formed by 65 observations) and a validation subset 608 (e.g., formed by 27 observations). The optimization procedure is repeated a total of 15 times using different subsets (randomly selected each time).

The mean of Matthews correlation coefficient (MCC) metric for each derived feature analysed and each combinations of m (equal to 1, 2 and 3), r (equal to 0.1, 0.2, 0.3 and 0.4 times the SD of each sliding window), and Th (15-50%) are calculated 610 in each repetition. The calculation of MCC involves values of all four elements of a confounding matrix, and it is considered a balanced measure, begin widely used in biomedical research applications. MCC is a correlation coefficient defined in terms of True Positive (TP), True Negative (TN), False Positive (FP) and False Negative (FN) values, ranging from -1 to +1. MCC is defined mathematically as follows:

$$MCC = \frac{TP*TN-FP*FN}{\sqrt{(TP+FP)*(TP+FN)*(TN+FP)*(TN+FN)}} \qquad (6)$$

An MCC value of -1 suggests a perfect disagreement between the objective and the predictions, 1 suggests a perfect agreement between the objective and the predictions, while 0 indicates that the prediction can be random with respect to the real data. The MCC index is used as the measure of effectiveness during the SE setting optimization process for robust CP-VI estimation.

A total of 15 repetitions are done using different subsets randomly selected each time. For each SE variable considered (SE-Flow$_{sum}$, SE-Flow$_{mean}$, SE-Flow$_{max}$, SE-Paw$_{sum}$, SE-Paw$_{mean}$, and SE-Paw$_{max}$) a maximum mean MCC value 614 is obtained.

The global maximum mean MCC value determines the optimal parameters 616 (m*, r* and Th*) among all possible combinations and all SE derived features. Finally, the mean behaviour for the optimal combination of parameters is shown, in both the optimization and the validation subsets (optimization performance 618, validation performance 620).

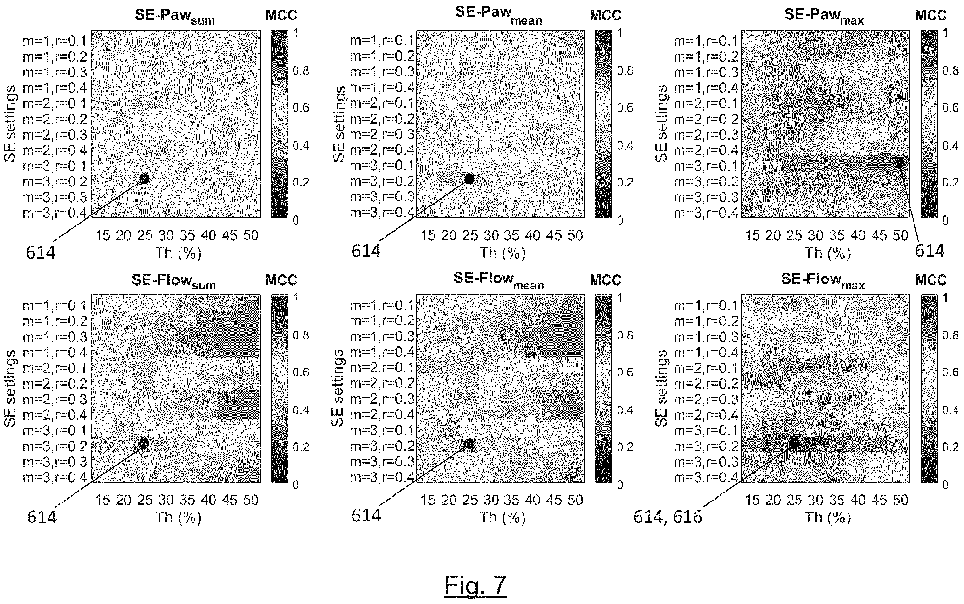

The graphs of **Figure 7** show the results of optimization process for SE-Paw (upper graphs) and for SE-Flow (lower graphs). Greyscale bar in each subplot shows the MCC mean scale, where values near to 1 represents more robust and consistent results. MCC metric reported positive values in all cases. The maximum mean MCC value 614 reached in each subplot is represented by a filled black circle. The highest average MCC values can be observed when using the feature "maximum value" for both SE-Paw and SE-Flow (i.e. SE-Flow$_{max}$ and SE-Paw$_{max}$). The highest MCC values are concentrated in values of m equal 2 and 3, r equal 0.1 and 0.2, and Th between 20% and 40%. In general, for all derived variables, the optimal SE settings are m=3, r=0.2 and Th=25% (except for SE-Paw$_{Max}$ with optimal settings m=3, r=0.1 and Th=50%, as represented by the filled black circle). Considering the optimal SE settings, the highest global maximum mean MCC value 616 (MCC=0.84) is obtained in this example for SE-Flow$_{Max}$ at Th=25% (SE-Flow$_{max}$25). In addition, in the example SE-Flow$_{max}$25 variable reached maximum MCC values in 13 times out of a total of 15 repetitions performed.

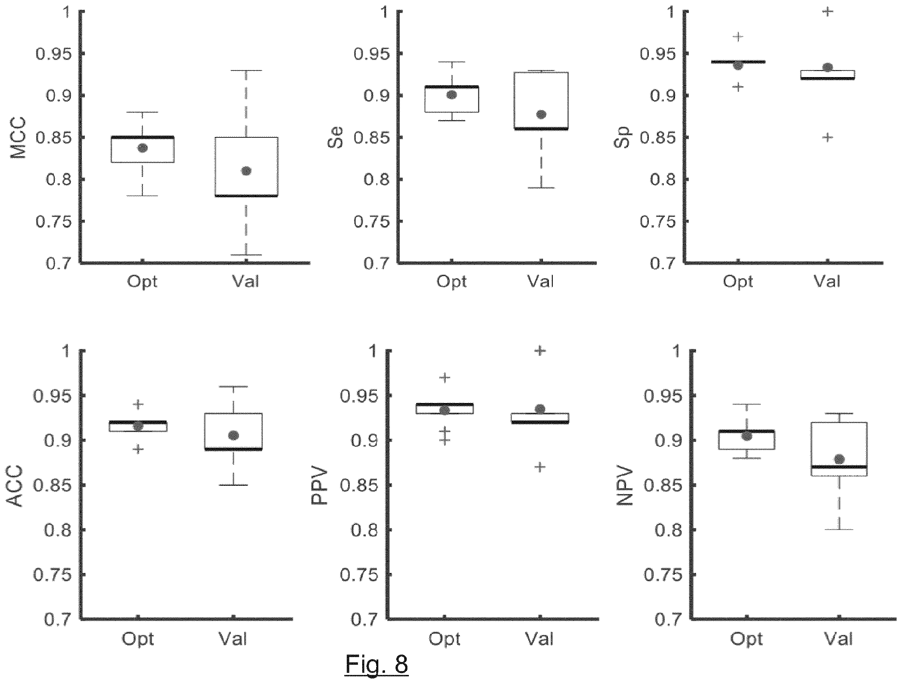

Once the feature that better reflects the CP-VI events is determined, different metrics are used to evaluate the algorithm performance on the 15 repetitions of the cross-validation procedure. The performance statistics obtained with the optimized parameters are shown in **Figure 8.** In particular, Figure 8 depicts performance statistics of $SE$-Flow$_{max}$25 in detecting CP-VI, including boxplot representation of Matthews correlation coefficient (MCC), sensitivity (Se), specificity (Sp), accuracy (ACC), positive and negative predictive values (PPV and NPV, respectively) for $SE$-Flow$_{max}$25 (m=3 and r=0.2xSD) corresponding to 15 repetitions during optimization and validation procedure, wherein the mean value is represented by a filled circle. In this example, $SE$-Flow$_{Max}$25 was able to detect CP-VI during optimization with MCC=0.85 (0.82-0.85), sensitivity= 0.91 (0.88-0.91), specificity=0.94 (0.94-0.94), accuracy=0.92 (0.91-0.92), PPV=0.94 (0.93-0.94) and NPV=0.91 (0.89-0.91). During validation, $SE$-Flow$_{Max}$25 detected CP-VI with MCC=0.78 (0.78-0.85), sensitivity=0.86 (0.86-0.93), specificity=0.92 (0.92-0.93), accuracy=0.89 (0.89-0.93), PPV=0.92(0.92-0.93) and NPV=0.87 (0.86-0.92). Observed median values of all features were slightly higher for optimization procedure than validation procedure, as a common consequence of the repeated holdout cross-validation process. The performance of $SE$-Flow$_{max}$25 according to ventilatory modes were consistent; for instance, in PSV, sensitivity=0.99, specificity=0.87, accuracy=0.93, and in assist-control modes, sensitivity=0.83, specificity=0.96, accuracy=0.89.

**Claims**

1. A device for respiratory monitoring in mechanically ventilated patients, comprising:

    an input unit (120) for receiving at least one respiratory signal (112) comprising an airway pressure signal (106) and/or a flow signal (108) acquired during mechanical ventilation of a patient (102);
    a data processing unit (130) configured to:

        for each respiratory signal (112), calculate (204) the sample entropy values (205) of a plurality of time-series data sets derived from the respiratory signal (112) to obtain (206) a sample entropy signal (208);
        extract (210) at least one feature (212) from each sample entropy signal (208);
        compare (214) the at least one feature (212) with a baseline condition (216); and
        detect (218) a change in the breathing pattern and/or clusters of asynchronies based on the comparison.

2. The device of claim 1, wherein the data processing unit (130) is configured to extract features (212) from each sample entropy signal (208) over a plurality of analysis time intervals (404).

3. The device of claim 2, wherein the data processing unit (130) is configured to assign the baseline condition (216) to the at least one feature (212) extracted on a first analysis time interval (404).

4. The device of claim 3, wherein the data processing unit (130) is configured to:
    for each analysis time interval (404), if at least one feature (212) extracted on the analysis time interval (404) is lower than the corresponding feature assigned to the baseline condition (216), update the baseline condition (216) with said lower extracted feature (212).

5. The device of any preceding claim, wherein the data processing unit (130) is configured to acquire the plurality of time-series data sets derived from each respiratory signal (112) using time sliding windows (402).

6. The device of any preceding claim, wherein the data processing unit (130) is configured to obtain each sample entropy signal (208) by applying smoothing on the corresponding sample entropy values (205).

7. The device of any preceding claim, wherein the at least one feature (212) extracted from each sample entropy signal (208) comprises any of the following:

    a maximum value;
    a mean value;
    a cumulative sum value; or
    a combination thereof.

8. The device of any preceding claim, wherein the data processing unit (130) is configured to:
    for each extracted feature (212), calculate an increase in the extracted feature relative to a corresponding feature of the baseline condition (216), and detect a change in the breathing pattern and/or clusters of asynchronies if the increase exceeds a threshold (Th).

9. The device of claim 8, wherein the respiratory signal (112) comprises a flow signal (108); wherein the sample entropy values (205) are calculated using an embedding dimension m equal to 3 and a tolerance $r$ comprised in the interval [0.1xSD-0.3xSD]; wherein the at least one feature (212) extracted from the sample entropy signal (208) associated to the flow signal (108) is the maximum value; and wherein the threshold (*Th*) is comprised in the interval [20%-30%].

10. The device of claim 8, wherein the respiratory signal (112) comprises an airway pressure signal (106); wherein the sample entropy values (205) are calculated using an embedding dimension m equal to 3 and a tolerance $r$ comprised in the interval [0.1xSD-0.2xSD]; wherein the at least one feature (212) extracted from the sample entropy signal (208) associated to the airway pressure signal (106) is the maximum value; and wherein the threshold (*Th*) is comprised in the interval [40%-50%].

11. The device of any preceding claim, further comprising an alarm unit (150) for emitting an alarm (222) when a change in the breathing pattern and/or clusters of asynchronies is detected.

12. A method for respiratory monitoring in mechanically ventilated patients, comprising:

receiving at least one respiratory signal (112) comprising an airway pressure signal (106) and/or a flow signal (108) acquired during mechanical ventilation of a patient (102); for each respiratory signal (112), calculating (204) the sample entropy values (205) of a plurality of time-series data sets derived from the respiratory signal (112), obtaining (206) a sample entropy signal (208); extracting (210) at least one feature (212) from each sample entropy signal (208); comparing (214) the at least one feature (212) with a baseline condition (216); and detecting (218) a change in the breathing pattern and/or clusters of asynchronies based on the comparison.

13. A computer program product for respiratory monitoring in mechanically ventilated patients, wherein the computer program product comprises at least one computer-readable storage medium comprising a set of instructions stored therein which, when executed by a processor, causes the processor to:

receive at least one respiratory signal (112) comprising an airway pressure signal (106) and/or a flow signal (108) acquired during mechanical ventilation of a patient (102); for each respiratory signal (112), calculate (204) the sample entropy values (205) of a plurality of time-series data sets derived from the respiratory signal (112), obtaining (206) a sample entropy signal (208); extract (210) at least one feature (212) from each sample entropy signal (208); compare (214) the at least one feature (212) with a baseline condition (216); and detect (218) a change in the breathing pattern and/or clusters of asynchronies based on the comparison.

PATIENT
102

MECHANICAL
VENTILATOR
104

PAW
SIGNAL
106

FLOW
SIGNAL
108

DATA
ACQUISITION
SYSTEM
110

RESPIRATORY
SIGNAL(S)
112

RESPIRATORY MONITORING DEVICE 100

WIRELESS
COMMUNICATION
UNIT 160

DISPLAY
170

INPUT UNIT 120

DATA PROCESSING UNIT 130

MECHANICAL
VENTILATOR
CONTROLLER
180

MEMORY 140

ALARM UNIT
150

EP 3 839 972 A1

Fig. 1

Fig. 2

Respiratory signals 112 → **Decimated** 202 → **SE (m,r) 30s sliding window, 50% overlap** 204 → **SE Smoothing** 206

**SE feature over 15-minute intervals** 210 → **PC from patient's own baseline SE** 214 → **PC > Th** 218 → CP-VI

EP 3 839 972 A1

Fig. 3

Fig. 4

No occurrence of CP-VI

CP-VI with return to baseline values

Progressive increase in CP-VI leading to self-extubation

Fig. 5A

Fig. 5B

Fig. 5C

EP 3 839 972 A1

Fig. 6

Fig. 7

Fig. 8

Europäisches
Patentamt
European
Patent Office
Office européen
des brevets

## EUROPEAN SEARCH REPORT

Application Number

EP 19 38 3116

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2016/279361 A1 (MULQUEENY QESTRA CAMILLE [AU] ET AL) 29 September 2016 (2016-09-29) * paragraph [0006] - paragraph [0021] * * paragraph [0063] - paragraph [0064] * * paragraph [0080] - paragraph [0089] * * paragraph [0122] * * paragraph [0163] - paragraph [0165] * * paragraph [0228] - paragraph [0229] * * paragraph [0271] - paragraph [0302] * * figures 1,12,14-16 * | 1-13 | INV. G16H50/20 A61B5/085 A61M16/00 |
| Y | US 2018/280646 A1 (FREEMAN JENNY [US] ET AL) 4 October 2018 (2018-10-04) * abstract * * paragraph [0007] - paragraph [0010] * * paragraph [0032] - paragraph [0033] * * paragraph [0097] * * paragraph [0216] - paragraph [0217] * * paragraph [0236] - paragraph [0243] * * paragraph [0262] - paragraph [0266] * * claims 1-3 * * figure 41 * | 1-13 | |
| A | RICHMAN J S ET AL: "Physiological time-series analysis using approximate entropy and sample entropy", AMERICAN JOURNAL OF PHYSIOLOGY, AMERICAN PHYSIOLOGICAL SOCIETY, US, vol. 278, no. 6 Part 2, 1 June 2000 (2000-06-01), pages H2039-H2049, XP008094440, ISSN: 0002-9513 * the whole document * | 1-13 | TECHNICAL FIELDS SEARCHED (IPC) G16H A61M A61B |

The present search report has been drawn up for all claims

| Place of search | Date of completion of the search | Examiner |
|---|---|---|
| Munich | 29 May 2020 | Hilbig, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 38 3116

This annex lists the patent family members relating to the patent documents cited in the above-mentioned European search report.
The members are as contained in the European Patent Office EDP file on
The European Patent Office is in no way liable for these particulars which are merely given for the purpose of information.

29-05-2020

| Patent document cited in search report | Publication date | Patent family member(s) | Publication date |
|---|---|---|---|
| US 2016279361 A1 | 29-09-2016 | EP 2421589 A1 | 29-02-2012 |
| | | EP 3351170 A1 | 25-07-2018 |
| | | JP 5639152 B2 | 10-12-2014 |
| | | JP 6246696 B2 | 13-12-2017 |
| | | JP 2012524552 A | 18-10-2012 |
| | | JP 2015037596 A | 26-02-2015 |
| | | JP 2017029792 A | 09-02-2017 |
| | | US 2012037159 A1 | 16-02-2012 |
| | | US 2016279361 A1 | 29-09-2016 |
| | | WO 2010121313 A1 | 28-10-2010 |
| US 2018280646 A1 | 04-10-2018 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **RICHMAN, J. S. ; MOORMAN, J. R.** Physiological time-series analysis using approximate entropy and sample entropy. *American Journal of Physiology - Heart and Circulatory,* 2000